# EUROPÄISCHE PATENTSCHRIFT

(11) **EP 0 968 169 B1**
(45) Veröffentlichungstag und Bekanntmachung des Hinweises auf die Patenterteilung: **22.05.2002**
(21) Anmeldenummer: 98906939.8
(22) Anmeldetag: 09.02.1998
(51) Int. Cl.: C07C 65/24

(54) **VERFAHREN ZUR ISOLIERUNG VON 3-[2-CHLOR-4-(TRIFLUORMETHYL)-PHENOXY]-BENZOESÄURE**
METHOD FOR ISOLATING 3-[2-CHLORO-4-(TRIFLUOROMETHYL)-PHENOXY]-BENZOIC ACID
PROCEDE PERMETTANT D'ISOLER DE L'ACIDE 3-[2-CHLORO-4-TRIFLUOROMETHYL)-PHENOXY]-BENZOIQUE

(30) Priorität: 21.02.1997 DE 19706875
(43) Veröffentlichungstag der Anmeldung: 05.01.2000
(73) Patentinhaber: BASF AKTIENGESELLSCHAFT, 67056 Ludwigshafen (DE)
(72) Erfinder: MERKLE, Hans, Rupert, D-67061 Ludwigshafen (DE); EVA, Ronald, R., Bahama, NC 79053 (US); FRANKE, Dirk, D-67067 Ludwigshafen (DE); JONES, Simon, A., D-69181 St. Ilgen (DE); MATTMANN, Wolfgang, D-67117 Limburgerhof (DE); MUNZINGER, Manfred, D-67246 Dirmstein (DE)
(86) Internationale Anmeldenummer: EP9800705
(87) Internationale Veröffentlichungsnummer: WO9837054

(56) Entgegenhaltungen:
- US-A- 4 031 131

## Beschreibung

Die vorliegende Erfindung betrifft ein Verfahren zur Isolierung von 3-[2-Chlor-4-(trifluormethyl)-phenoxy]-benzoesäure der Formel I 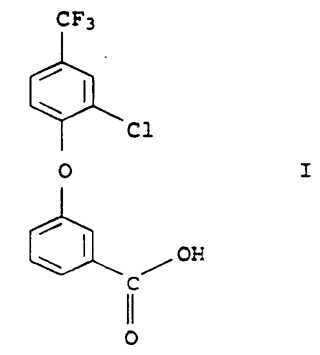 aus einer wässrigen Lösung seines Metallsalzes, bei dem man
a) das Metallsalz der 3-[2-Chlor-4-(trifluormethyl)-phenoxy]-benzoesäure mit einer organischen oder anorganischen Säure umsetzt;
b) das Reaktionsgemisch erhitzt, bis sich ein flüssiges Zweiphasensystem aus organischer und wässriger Phase gebildet hat; und
c) die organische Phase von der wässrigen Phase abtrennt.

3-[2-Chlor-4-(trifluormethyl)-phenoxy]-benzoesäure der Formel I ist ein Zwischenprodukt für die Herstellung der Herbizide Acifluorfen, Fluoroglycofen und Lactofen, deren Herstellung in der DE-A 23 11 638, EP-A 20 052, DE-A 30 29 728 und US 4 031 131 beschrieben ist.

Gemäß der Beschreibung der US 4 031 131 wird das Benzoesäure-Derivat der Formel I aus seiner Salzlösung durch Neutralisation mit einer anorganischen Säure als Kristallisat ausgefällt, durch Filtration isoliert, mit Wasser gewaschen und getrocknet.

Dieses Verfahren hat einige Nachteile: Das Handling des Feststoffes führt zur Krustenbildung bei der Kristallisation, wobei die Kristalle deutlich mehr Wasser aufnehmen. Diese Eigenschaft führt beim Trocknen zu Verstopfungen und zu Verfahrensengpässen.

Aufgabe der vorliegenden Erfindung war es, ein Verfahren zur Isolierung des Benzoesäure-Derivats der Formel I zur Verfügung zu stellen, das diese Nachteile nicht aufweist.

Diese Aufgabe wurde erfindungsgemäß durch das eingangs beschriebene Verfahren gelöst.

Die Herstellung des Benzoesäure-Derivates der Formel I erfolgt nach der US 4 031 131 nach folgendem Schema:

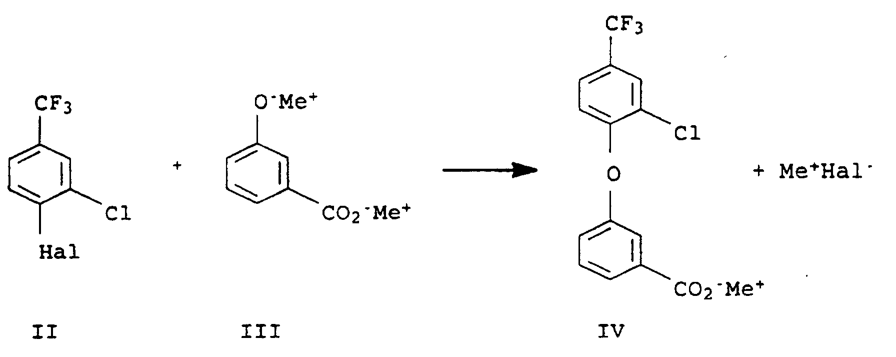

Das Trifluormethylbenzol-Derivat der Formel II wird mit dem Dimetallsalz der 3-Hydroxybenzoesäure zum Metallsalz des Benzoesäure-Derivats der Formel IV umgesetzt.

Die Isolierung der freien Säure des Benzoesäure-Derivats der allgemeinen Formel I erfolgt nach dem erfindungsgemäßen Verfahren in drei Stufen.

In der Verfahrensstufe a) wird die wäßrige Lösung der Metallsalze des Benzoesäure-Derivats der Formel I mit einer organischen oder anorganischen Säure umgesetzt.

Prinzipiell kann von allen Metallsalzen Me⁺, einwertigen und mehrwertigen, der Verbindung der Formel IV ausgegangen werden, wobei die wasserlöslichen bevorzugt sind.

Von den wasserlöslichen sind die Alkalisalze bevorzugt. Besonders bevorzugt sind Salze des Kaliums und Natriums.

Zur Neutralisation der Metallsalze der Formel IV sind alle Ansprüche und anorganischen Säuren geeignet. Beispiele für organische Säuren sind Ameisensäure, Essigsäure, Propionsäure und deren Gemische. Bevorzugt wird Essigsäure verwendet.

Geeignete anorganische Säuren sind Salzsäure, Schwefelsäure, Salpetersäure, Phosphorsäure und deren Gemische.

Die organische oder anorganische Säure wird zu der wäßrigen Lösung des Metallsalzes der Formel IV unter rühren bei einer Temperatur von 20 bis 127°C, bevorzugt 20 bis 115°C gegeben, bis ein pH von kleiner 5, bevorzugt kleiner 4,5 erreicht ist. Das entspricht etwa der 1- bis 6-fachen molaren Menge Säure pro Mol Metallsalz der Formel IV, bevorzugt 1- bis 5-fachen molaren Menge Säure. Liegen die Temperaturen unter etwa 90°C, fällt das Benzoesäure-Derivat I aus.

In der Stufe b) wird das Reaktionsgemisch erhitzt, bis das Benzosäure-Derivat I geschmolzen ist und sich ein flüssiges Zweiphasensystem aus organischer Phase und wässriger Phase gebildet hat. Die untere organische Phase enthält das geschmolzene Benzoesäure-Derivat I. Die Temperaturen liegen dabei zwischen 90 und 127°C, bevorzugt 95 bis 115°C.

Die Stufen a) und b) können auch in einer Reaktionsstufe durchgeführt werden, indem die Zugabe der Säure bei einer Temperatur zwischen 90 und 127°C, bevorzugt 95 bis 115°C durchgeführt wird. Bei dieser Reaktionsführung entsteht unmittelbar ein flüssiges Zweiphasensystem, das sich in eine untere organische Phase und eine obere wäßrige Phase trennt.

In der Stufe c) wird die organische Phase von der wässrigen Phase getrennt. Die organische Phase enthält das Benzoesäure-Derivat, Wasser und ggf. organische Säure.

Die organische Phase kann unmittelbar für die Weiterverarbeitung verwendet werden.

In der Stufe d) kann sie aber noch vorher entwässert werden. Dies geschieht durch Abdestillation des Wassers und der Reste organischer Säure. Für den Fall der Verwendung von Essigsäure als organische Säure bei der Neutralisationsstufe a) kann das Wasser auch durch Abdestillation von Teilen oder der Gesamtmenge an Essigsäure entfernt werden.

Das erfindungsgemäße Verfahren kann kontinuierlich und diskontinuierlich sowie bei Normaldruck, im Überdruck und Unterdruck durchgeführt werden.

Die Ausbeute an 3-[2-Chlor-4-(trifluormethyl)-phenoxy]-benzoesäure in der organischen Phase bei dem erfindungsgemäßen Verfahren ist sehr hoch. Die abgetrennte wässrige Phase enthält weniger als 3 %, bevorzugt weniger als 2 % dieser Verbindung.

Das Feststoffhandling entfällt bei diesem Isolierverfahren und die entwässerte Schmelze der 3-[2-Chlor-4-(trifluormethyl)-phenoxy]-benzoesäure kann unmittelbar zur Herstellung von Acifluorfen, 5-[2-Chlor-4-(trifluormethyl)-phenoxy]-2-nitrobenzoesäure, durch Nitrierung weiterverwendet werden.

### Beispiel 1:

2500 Teile einer 20 Gew.-%igen Kaliumsalz-Lösung (bezogen auf freie Säure) von 3-[2-Chlor-4-(trifluormethyl)-phenoxy]-benzoesäure (1,58 Mol) von pH 8,8 werden in einem heizbaren 3-Liter Glasgefäß unter Rühren mit 498 Teilen Wasser verdünnt und auf 50°C erwärmt. Anschließend werden bei dieser Temperatur unter Rühren innerhalb von 90 Minuten 370 Teile (6,17 Mol) Essigsäure zugeführt, wobei 3-[2-Chlor-4-(trifluormethyl)-phenoxy]-benzoesäure ausfällt. Der pH der resultierenden Suspension beträgt 4,3. Danach wird die Suspension auf 96°C erwärmt, wobei der Feststoff schmilzt und sich ein zweiphasiges flüssiges Gemisch bildet. Nach Abstellen des Rührers trennen sich die Phasen; die untere organische braune Schmelzphase wiegt nach Abtrennung der wässrigen Phase 594 Teile und enthält neben 84,0 Gew.-% 3-[2-Chlor-4-(trifluormethyl)-phenoxy]-benzoesäure noch 12,9 Gew.-% Wasser sowie 2,33 Gew.-% Acetat als freie Säure bzw. Kaliumsalz. Die wässrige Phase enthält 3-[2-Chlor-4-(tri-fluormethyl)-phenoxy]-benzoesäure nur zu < 0,1 %.

### Beispiel 2:

2500 Teile einer 20 Gew.-%igen Natriumsalz-Lösung (bezogen auf freie Säure) von 3-[2-Chlor-4-(trifluormethyl)-phenoxy]-benzoesäure (1,58 Mol) von pH 8,8 werden in einem heizbaren 3-Liter Glasgefäß unter Rühren mit 498 Teilen Wasser verdünnt und auf 50°C erwärmt. Anschließend werden bei dieser Temperatur unter Rühren innerhalb von 90 Minuten 360 Teile (6,0 Mol) Essigsäure zugeführt, wobei 3-[2-Chlor-4-(trifluormethyl)-phenoxy]-benzoesäure ausfällt. Der pH der resultierenden Suspension beträgt 4,3. Danach wird die Suspension auf 100°C erwärmt, wobei der Feststoff schmilzt und sich ein zweiphasiges flüssiges Gemisch bildet. Nach Abstellen des Rührers trennen sich die Phasen; die untere organische braune Schmelzphase wiegt nach Abtrennung der wässrigen Phase 485 Teile und enthält neben 82,5 Gew.-% 3-[2-Chlor-4-(trifluormethyl)-phenoxy] - benzoesäure noch 14,5 Gew.-% Wasser sowie 2,5 Gew.-% Acetat als freie Säure bzw. Natriumsalz. Die wässrige Phase enthält 3-[2-Chlor-4-(tri-fluormethyl)-phenoxy]-benzoesäure nur zu < 0,1 %.

## Patentansprüche

1. Verfahren zur Isolierung von 3-[2-Chlor-4-(trifluormethyl)-phenoxy]-benzoesäure der Formel I 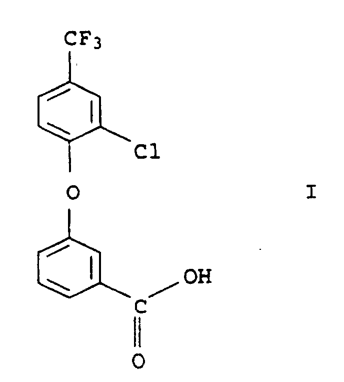 aus einer wässrigen Lösung seines Metallsalzes, **dadurch gekennzeichnet, daß** man
a) das Metallsalz der 3-[2-Chlor-4-(trifluormethyl)-phenoxy]-benzoesäure mit einer organischen oder anorganischen Säure umsetzt;
b) das Reaktionsgemisch erhitzt, bis sich ein flüssiges Zweiphasensystem aus organischer und wässriger Phase gebildet hat; und
c) die organische Phase von der wässrigen Phase abtrennt.

2. Verfahren nach Anspruch 1, **dadurch gekennzeichnet, daß** man die Stufen a) und b) in einer Reaktionsstufe zusammenfaßt.

3. Verfahren nach einem der Ansprüche 1 oder 2, **dadurch gekennzeichnet, daß** man in einer weiteren Stufe d) die organische Phase der Stufe c) entwässert.

4. Verfahren nach einem der Ansprüche 1 bis 3, **dadurch gekennzeichnet, daß** als Metallsalz ein Natrium- oder Kaliumsalz verwendet.

5. Verfahren nach einem der Ansprüche 1 bis 4, **dadurch gekennzeichnet, daß** man als organische Säure Ameisensäure, Essigsäure, Propionsäure sowie deren Gemische verwendet.

6. Verfahren nach Anspruch 5, **dadurch gekennzeichnet, daß** man als organische Säure Essigsäure verwendet.

7. Verfahren nach einem der Ansprüche 1 bis 4, **dadurch gekennzeichnet, daß** man als anorganische Säure Salzsäure, Schwefelsäure, Phosphorsäure, Salpetersäure oder deren Gemische verwendet.

8. Verfahren nach einem der Ansprüche 1 bis 7, **dadurch gekennzeichnet, daß** man in der Stufe a) soviel organische oder anorganische Säure zugibt, bis ein pH von kleiner 5 erreicht ist.

9. Verfahren nach einem der Ansprüche 1 bis 8, **dadurch gekennzeichnet, daß** man das Reaktionsgemisch in der Stufe b) auf mindestens 90°C erhitzt.

10. Verfahren nach Anspruch 9, **dadurch gekennzeichnet, daß** man das Reaktionsgemisch auf mindestens 96°C erhitzt.

11. Verfahren nach einem der Ansprüche 3 bis 10, **dadurch gekennzeichnet, daß** man in der Stufe d) die organische Phase durch Abdestillation des Wassers entwässert.

12. Verfahren nach Anspruch 6, **dadurch gekennzeichnet, daß** man in der Stufe d) die organische Phase durch Abdestillation von Essigsäure entwässert.

## Claims

1. A process for the isolation of 3-[2-chloro-4-(trifluoromethyl)phenoxy]benzoic acid of the formula I 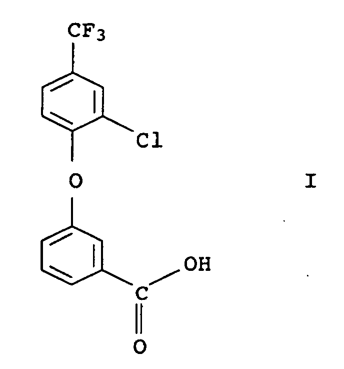 from an aqueous solution of its metal salt, which comprises
a) reacting the metal salt of 3-[2-chloro-4-(trifluoromethyl)phenoxy]benzoic acid with an organic or inorganic acid;
b) heating the reaction mixture until a liquid two-phase system of organic and aqueous phase has formed; and
c) separating the organic phase from the aqueous phase.

2. The process as claimed in claim 1, wherein steps a) and b) are combined in one reaction step.

3. The process as claimed in claim 1 or 2, wherein the organic phase of step c) is dewatered in an additional step d).

4. The process as claimed in any of claims 1 to 3, wherein the metal salt used is a sodium or potassium salt.

5. The process as claimed in any of claims 1 to 4, wherein the organic acid used is formic acid, acetic acid, propionic acid or mixtures thereof.

6. The process as claimed in claim 5, wherein the organic acid used is acetic acid.

7. The process as claimed in any of claims 1 to 4, wherein the inorganic acid used is hydrochloric acid, sulfuric acid, phosphoric acid, nitric acid or mixtures thereof.

8. The process as claimed in any of claims 1 to 7, wherein sufficient organic or inorganic acid is added in step a) to obtain a pH of less than 5.

9. The process as claimed in any of claims 1 to 8, wherein the reaction mixture in step b) is heated to at least 90°C.

10. The process as claimed in claim 9, wherein the reaction mixture is heated to at least 96°C.

11. The process as claimed in any of claims 3 to 10, wherein the organic phase is dewatered in step d) by distilling off the water.

12. The process as claimed in claim 6, wherein the organic phase is dewatered in step d) by distilling off acetic acid.

## Revendications

1. Procédé pour isoler l'acide 3-[2-chloro-4-(trifluorométhyl)-phénoxy]benzoïque de formule I 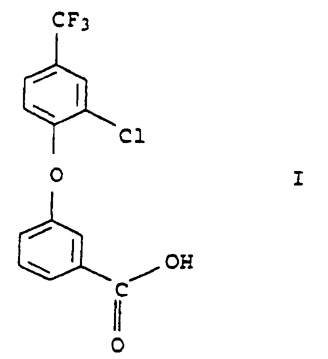 d'une solution aqueuse de l'un de ses sels métalliques, **caractérisé par le fait que**
a) on fait réagir le sel métallique de l'acide 3-[2-chloro-4-(trifluorométhyl)phénoxy]-benzoïque avec un acide organique ou minéral ;
b) on chauffe le mélange de réaction jusqu'à ce qu'il se forme un système liquide à deux phases consistant en une phase organique et une phase aqueuse ; et
c) on sépare la phase organique de la phase aqueuse.

2. Procédé selon la revendication 1, **caractérisé par le fait que** les opérations a) et b) sont combinées en un seul stade de réaction.

3. Procédé selon l'une des revendications 1 ou 2, **caractérisé par le fait que**, dans un autre stade opératoire d), on déshydrate la phase organique obtenue au stade c).

4. Procédé selon l'une des revendications 1 à 3, **caractérisé par le fait que** le sel métallique utilisé est un sel de sodium ou de potassium.

5. Procédé selon l'une des revendications 1 à 4, **caractérisé par le fait que** l'acide organique utilisé est l'acide formique, l'acide acétique, l'acide propionique ou un mélange de ces acides.

6. Procédé selon la revendication 5, **caractérisé par le fait que** l'acide organique utilisé est l'acide acétique.

7. Procédé selon l'une des revendications 1 à 4, caracérisé par le fait que l'acide minéral utilisé est l'acide chlorhydrique, l'acide sulfurique, l'acide phosphorique, l'acide nitrique ou un mélange de ces acides.

8. Procédé selon l'une des revendications 1 à 7, **caractérisé par le fait que**, au stade a), on ajoute un acide organique ou minéral jusqu'à pH inférieur à 5.

9. Procédé selon l'une des revendications 1 à 8, **caractérisé par le fait que**, au stade b), on chauffe le mélange de réaction à une température d'au moins 90°C.

10. Procédé selon la revendication 9, **caractérisé par le fait que** l'on chauffe le mélange de réaction à au moins 96°C.

11. Procédé selon l'une des revendications 3 à 6, **caractérisé par le fait que**, au stade d), on déshydrate la phase organique par distillation de l'eau.

12. Procédé selon la revendication 6, **caractérisé par le fait que**, au stade d), on déshydrate la phase organique par distillation de l'acide acétique.
